(19) Europäisches Patentamt
European Patent Office
Office européen des brevets

(11) EP 0 797 980 A1

(12) **EUROPÄISCHE PATENTANMELDUNG**

(43) Veröffentlichungstag:
**01.10.1997 Patentblatt 1997/40**

(51) Int. Cl.[6]: **A61K 7/13**, C07C 255/58

(21) Anmeldenummer: **96119746.4**

(22) Anmeldetag: **10.12.1996**

(84) Benannte Vertragsstaaten:
**DE ES FR GB IT**

(30) Priorität: **29.03.1996 DE 19612506**

(71) Anmelder: **Wella Aktiengesellschaft**
**64274 Darmstadt (DE)**

(72) Erfinder:
- **Keller, Helmut**
  **64287 Darmstadt (DE)**
- **Balzer, Wolfgang R., Dr.**
  **64665 Alsbach (DE)**

(54) **Oxidationshaarfärbemittel mit einen Gehalt von 2,5-Diaminobenzonitrilen sowie neue 2,5-Diaminobenzonitrile**

(57) Mittel zur oxidativen Färbung von Haaren auf der Basis einer Entwicklersubstanz-Kupplersubstanz-Kombination, welche als Entwicklersubstanz ein 2,5-Diaminobenzonitril der allgemeinen Formel (I)

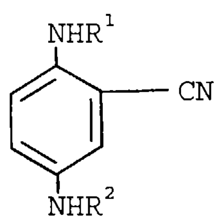

(I),

in der $R^1$ und $R^2$ unabhängig voneinander gleich Wasserstoff, Alkyl mit 1 bis 6 Kohlenstoffatomen oder Hydroxyalkyl mit 2 bis 4 Kohlenstoffatomen oder Dihydroxyalkyl mit 3 oder 4 Kohlenstoffatomen sind, unter der Voraussetzung, daß die beiden Reste $R^1$ und $R^2$ nicht gleichzeitig Wasserstoff darstellen, oder dessen physiologisch verträgliches, wasserlösliches Salz enthält, sowie neue 2,5-Diaminobenzonitrile.

EP 0 797 980 A1

## Beschreibung

Gegenstand der Erfindung sind Mittel zur oxidativen Färbung von Haaren auf der Basis einer Entwicklersubstanz/Kupplersubstanz-Kombination, welche als Entwicklersubstanz 2,5-Diaminobenzonitrile enthalten sowie neue 2,5-Diaminobenzonitrile.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diaminotoluol, 2,5-Diaminophenylethylalkohol, p-Aminophenol und 1,4-Diaminobenzol eingesetzt. Von den vorzugsweise verwendeten Kupplersubstanzen sind Resorcin, 4-Chlorresorcin, 1-Naphthol, 3-Aminophenol und Derivate des m-Phenylendiamins zu nennen.

An Oxidationsfarbstoffe, die zur Färbung menschlicher Haare verwendet werden, werden zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure und Reibeechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwickler- und Kupplerkomponenten eine breite Palette verschiedener Farbnuancen erzeugt werden kann.

Mit den derzeit eingesetzten Haarfärbemitteln ist es jedoch nicht möglich, die vorgenannten Anforderungen in allen Punkten zu erfüllen, da insbesondere bei den verwendeten Entwicklersubstanzen Probleme bestehen.

Es bestand daher die Aufgabe, für die Verwendung in Oxidationshaarfärbemitteln auf der Basis einer Entwicklersubstanz/Kupplersubstanz-Kombination geeignete Entwicklersubstanzen zur Verfügung zu stellen.

Hierzu wurde nun gefunden, daß durch ein Mittel zur oxidativen Färbung von Haaren auf der Basis einer Entwicklersubstanz-Kupplersubstanz-Kombination, welche als Entwicklersubstanz ein 2,5-Diaminobenzonitril der allgemeinen Formel (I),

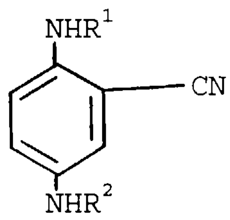

(I),

in der $R^1$ und $R^2$ unabhängig voneinander gleich Wasserstoff, Alkyl mit 1 bis 6 Kohlenstoffatomen, Hydroxyalkyl mit 2 bis 4 Kohlenstoffatomen oder Dihydroxyalkyl mit 3 oder 4 Kohlenstoffatomen sind, unter der Voraussetzung, daß die beiden Reste $R^1$ und $R^2$ nicht gleichzeitig Wasserstoff darstellen, oder dessen physiologisch verträgliche, wasserlösliche Salze enthält, diese Aufgabe in hervorragender Weise gelöst wird.

In dem Haarfärbemittel sollen die Entwicklersubstanzen der Formel (I), von denen das 5-Amino-2-methylamino-benzonitril, 5-Amino-2-(2'-hydroxyethyl)amino-benzonitril, 5-Amino-2-(2',3'-dihydroxypropyl)amino-benzonitril, 2-Amino-2-(2'-hydroxyethyl)amino-benzonitril und 2-Amino-2-(2',3'-dihydroxypropyl)amino-benzonitril sowie deren Hydrochloride bevorzugt sind, in einer für die Haarfärbung ausreichende Menge, im allgemeinen in einer Menge von 0,01 bis 5,0 Gewichtsprozent, vorzugsweise in einer Menge von 0,1 bis 3,5 Gewichtsprozent, enthalten sein.

Obwohl die vorteilhaften Eigenschaften der hier beschriebenen Entwicklersubstanzen der Formel (I) es nahelegen, diese als alleinige Entwicklersubstanz zu verwenden, ist es selbstverständlich auch möglich, die Entwicklersubstanzen der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol, 4-Amino-3-methylphenol, 4,5-Diaminopyrazolderivaten oder Tetraaminopyrimidinen, einzusetzen.

Als Kupplersubstanzen kommen vorzugsweise 1-Naphthol, Resorcin, 4-Chlorresorcin, 2-Methylresorcin, 2-Amino-4-(2'-hydroxyethyl)amino-anisol, 2,4-Diamino-5-ethoxytoluol, 4,6-Dichlorresorcin, m-Phenylendiamin,5-Amino-2-methylphenol, 2,4-Diaminophenoxyethanol, 4-Amino-2-hydroxyphenoxyethanol. 3-Aminophenol, 3-Amino-2-methylphenol, 4-Hydroxy-1,2-methylendioxybenzol, 4-Amino-1,2-methylendioxybenzol, 4-(2'-Hydroxyethyl)amino-1,2 -methylendioxybenzol, 2,4-Diamino-5-fluortoluol, 3-Amino-4-chlor-6-methylphenol, 3-Amino-2-chlor-6-methylphenol, 3-Amino-4-fluor-6-methylphenol, 4-Hydroxyindol, 3-Amino-4-methoxy-6-methylphenol und 3,5-Diamino-2,6-dimethoxypyridin in Betracht, wobei die Einsatzmenge der Kupplersubstanz etwa 0,01 bis 5,0 Gewichtsprozent, vorzugsweise 0,1 bis 2,5 Gewichtsprozent beträgt.

Die Kuppler- und Entwicklersubstanzen können mit dem Haarfärbemittel jeweils einzeln oder im Gemisch miteinander enthalten sein.

Die Gesamtmenge der in dem hier beschriebenen Haarfärbemittel enthaltenen Entwicklersubstanz-Kupplersubstanz-Kombination beträgt 0,02 bis 10,0 Gewichtsprozent, wobei eine Menge von 0,2 bis 6,0 Gewichtsprozent bevorzugt ist. Die Entwicklerkomponenten werden im allgemeinen in etwa äquimolaren Mengen, bezogen auf die Kupplerkomponenten, eingesetzt. Es ist jedoch nicht nachteilig, wenn die Entwicklerkomponenten diesbezüglich in einem gewissen Überschuß oder Unterschuß vorhanden sind.

Weiterhin kann das erfindungsgemäße Haarfärbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'-imino-2",5"-cyclohexadien-1"-yliden)-methyl]-2-methylaminobenzol-monohydrochlorid (C.I. 42 510) und 4-[(4'-amino-3'-methyl-phenyl)-(4"-imino-3"-methyl-2",5"-cyclohexadien-1"-yliden)-methyl]-2-methyl-aminobenzolmonohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol und 1-[(2'-Ureidoethyl)amino-4-nitrobenzol, Azofarbstoffe wie 6-[(4'-Aminophenyl)azo]-5-hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Haarfärbemittel können diese Farbkomponenten in einer Menge von etwa 0,1 bis 4,0 Gewichtsprozent enthalten.

Selbstverständlich können die Kuppler- und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem Haarfärbemittel noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Haarfärbemittels kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert von 6,8 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen zum Beispiel Phosphorsäure sowie Essigsäure oder andere organische Säuren, wie zum Beispiel Zitronensäure oder Weinsäure, in Betracht.

Für die Anwendung zur oxidativen Färbung von Haaren vermischt man das vorstehend beschriebene Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3- bis 12prozentigen, vorzugsweise 6prozentigen, wäßrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die in dem erfindungsgemäßen Haarfärbemittel enthaltenen 2,5-Diaminobenzonitrile der Formel (I) lassen sich beispielsweise nach dem folgenden Schema durch katalytische Hydrierung der entsprechenden 2-Amino-5-nitrobenzonitrile (III a) beziehungsweise 5-Amino-2-nitrobenzonitrile (III b) herstellen (wobei R die gleiche Bedeutung wie $R^1$

und $R^2$ hat):

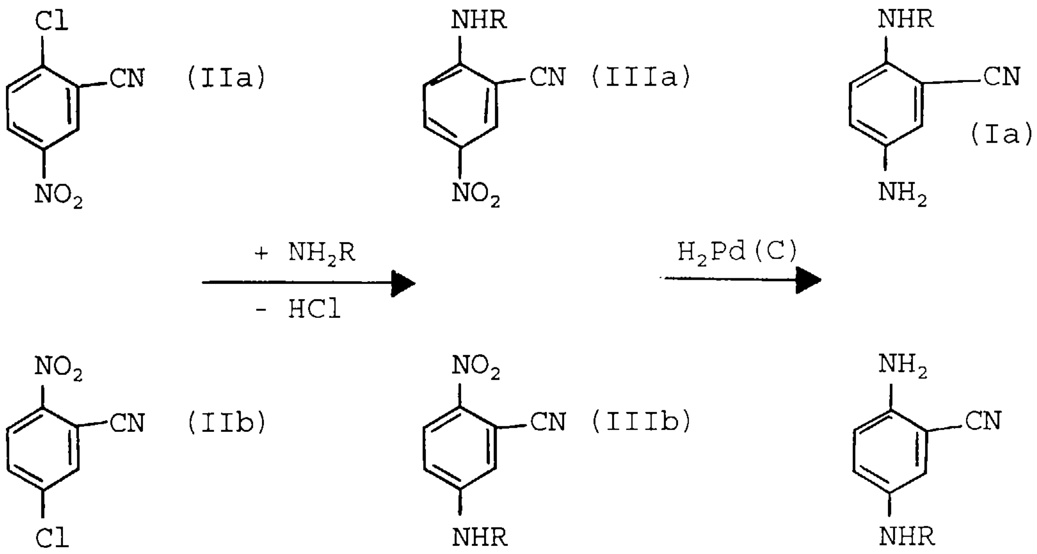


(IIa) (IIIa) (Ia)

(IIb) (IIIb)

Gegebenenfalls kann die Aminogruppe in 2- beziehungsweise 5-Stellung alkyliert werden.

Gegenstand der vorliegenden Patentanmeldung sind daher ferner die neuen 2,5-Diaminobenzonitrile der allgemeinen Formel (I), wobei insbesondere 5-Amino-2-methylamino-benzonitril, 2-Amino-5-(2'-hydroxyethyl)amino-benzonitril, 2-Amino-5-(2'3'-dihydroxypropyl)amino-benzonitril, 5-Amino-2-(2'-hydroxyethyl)amino-benzonitril und 5-Amino-2-(2'3'-dihydroxypropyl)amino-benzonitril zu nennen sind.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden. Die Verbindungen der Formen (I) sind gut in Wasser löslich und weisen zusätzlich eine ausgezeichnete Lagerstabilität, insbesondere als Bestandteil der hier beschriebenen Haarfärbemittel, auf.

Das erfindungsgemäße Haarfärbemittel mit einem Gehalt an 2,5-Diaminobenzonitrilen der Formel (I) als Entwicklersubstanz ermöglicht Haarfärbungen mit ausgezeichneter Farbechtheit, insbesondere was die Licht-, Wasch- und Reibeechtheit anbetrifft.

Hinsichtlich der färberischen Eigenschaften bietet das erfindungsgemäße Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus.

Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

**Beispiele**

**Herstellungsbeispiele**

**Beispiel 1:** Synthese von 2-Alkylamino-5-nitrobenzonitrilen

**Allgemeine Vorschrift:**

18,25 g (0,1 mol) 2-Chlor-5-nitrobenzonitril werden in 50 ml Ethanol gelöst. Anschließend werden unter Rückfluß 0,22 mol des entsprechenden Amins zugegeben. Nach beendeter Reaktion wird das Lösungsmittel am Rotationsverdampfer abdestilliert und der Rückstand mit 150 ml Eiswasser versetzt. Der erhaltene Niederschlag wird abfiltriert, zweimal mit 50 ml Eiswasser gewaschen und sodann getrocknet.

**1a. 2-Methylamino-5-nitro-benzonitril**

Verwendetes Amin: Methylamin
Ausbeute: 16,3 g (= 92 Prozent der Theorie)
Schmelzpunkt: 180° Celsius (gelbe Kristalle)

**1b. 2-(2'-Hydroxyethyl)amino-5-nitro-benzonitril**

Verwendetes Amin: 2-Hydroxyethylamin
Ausbeute: 19,8 g (= 96 Prozent der Theorie)
Schmelzpunkt: 158 ° Celsius (gelbe Kristalle)

**1c. 2-(2',3'-Dihydroxypropyl)amino-5-nitro-benzonitril**

Verwendetes Amin: 2,3-Dihydroxypropylamin
Ausbeute: 21,2 g (= 89 Prozent der Theorie)
Schmelzpunkt: 144° Celsius (gelbe Kristalle)

**Beispiel 2:** Synthese von 5-Alkylamino-2-nitrobenzonitrilen

**Allgemeine Vorschrift:**

18,25 g (0,1 mol) 5-Chlor-2-nitrobenzonitril werden in 50 ml Ethanol gelöst. Anschließend werden unter Rückfluß 0,22 mol des entsprechenden Amins zugegeben. Nach beendeter Reaktion wird das Lösungsmittel am Rotationsverdampfer abdestilliert und der Rückstand mit 150 ml Eiswasser versetzt. Der erhaltene Niederschlag wird abfiltriert, zweimal mit 50 ml Eiswasser gewaschen und sodann getrocknet.

**2a. 5-(2'-Hydroxyethyl)amino-2-nitro-benzonitril**

Verwendetes Amin: 2-Hydroxyethylamin
Ausbeute: 18,3 g (= 88 Prozent der Theorie)
Schmelzpunkt: 116° Celsius (gelbe Kristalle)

**2b. 5-(2',3'-Dihydroxypropyl)amino-2-nitro-benzonitril**

Verwendetes Amin: 2,3-Dihydroxypropylamin
Ausbeute: 20,5 g (85 Prozent der Theorie)
Schmelzpunkt: 136° Celsius (gelbe Kristalle)

**Beispiel 3:** Synthese von 2-Alkylamioo-5-amino-benzonitrilen und 5-Alkylamino-2-amino-benzonitrilen

**Allgemeine Vorschrift:**

10 mmol Alkylamino-amino-benzonitril gemäß Beispiel 1 oder 2 werden in 50 ml Ethanol gelöst und unter Zusatz von 50 mg eines Palladium-Aktivkohle-Katalysators (10%ig) bei 50° Celsius hydriert. Nach Aufnahme der erforderlichen Wasserstoffmenge wird vom Katalysator abfiltriert und mit einem Überschuß an verdünnter Salzsäure versetzt. Nach dem Einengen der Lösung am Rotationsverdampfer wird das ausgefallene Hydrochlorid abfiltriert und getrocknet.

**3a. 5-Amino-2-methylamino-benzonitril-hydrochlorid**

Schmelzpunkt: 230° Celsius (Zersetzung); farblose Kristalle

| CHN-Analyse: ($C_8H_{10}N_3Cl$) | % C | % H | % N |
|---|---|---|---|
| berechnet: | 52,32 | 5,49 | 22,88 |
| gefunden: | 52,49 | 5,53 | 22,75 |

**3b. 5-Amino-2-(2'-hydroxyethyl)amino-benzonitril-Hydrochlorid**

Schmelzpunkt: 153° Celsius (Zersetzung); farblose Kristalle

| CHN-Analyse: ($C_9H_{12}N_3OCl$) | % C | % H | % N |
|---|---|---|---|
| berechnet: | 50,59 | 5,66 | 19,67 |
| gefunden: | 49,98 | 5,74 | 19,67 |

**3c. 5-Amino-2-(2',3'-dihydroxypropyl)amino-benzonitril-Hydrochlorid**

Schmelzpunkt: 135° Celsius (Zersetzung); farblose Kristalle

| CHN-Analyse: ($C_{10}H_{14}N_3O_2Cl$) | % C | % H | % N |
|---|---|---|---|
| berechnet: | 49,28 | 5,79 | 17,25 |
| gefunden: | 49,15 | 5,96 | 17,08 |

**3d. 2-Amino-5-(2'-hydroxyethyl)amino-benzonitril-Hydrochlorid**

Schmelzpunkt: 160° Celsius (Zersetzung); farblose Kristalle

| CHN-Analyse: ($C_9H_{12}N_3OCl$) | % C | % H | % N |
|---|---|---|---|
| berechnet: | 50,59 | 5,66 | 19,67 |
| gefunden: | 50,05 | 6,02 | 18,90 |

**3e. 2-Amino-5-(2'3'-dihydroxypropyl)amino-benzonitril-Hydrochlorid**

Schmelzpunkt: 130° Celsius (Zersetzung); farblose Kristalle

| CHN-Analyse: ($C_{10}H_{14}N_3O_2Cl$) | % C | % H | % N |
|---|---|---|---|
| berechnet: | 49,28 | 5,79 | 17,25 |
| gefunden: | 49,25 | 5,77 | 17,75 |

**Beispiele für Haarfärbemittel**

**Beispiel 4 bis 15** Haarfärbemittel

Es werden Haarfärbelösungen der folgenden Zusammensetzung hergestellt:

| | |
|---|---|
| 0,025 mol | Entwickler gemäß Tabelle 1 |
| 0,025 mol | Kuppler gemäß Tabelle 1 |
| 25,0 g | Ölsäure |
| 10,0 g | Ammoniak (22prozentige wäßrige Lösung) |
| 7,0 g | Isopropanol |
| 0,3 g | Ascorbinsäure |
| ad 100,0 g | Wasser |

50 g der vorstehenden Haarfärbelösung werden kurz vor Gebrauch mit 50 g einer 6prozentigen Wasserstoffperoxidlösung vermischt und das Gemisch wird sodann auf gebleichte Haare aufgetragen. Nach einer Einwirkungszeit von 30 Minuten bei 40° Celsius wird das Haar mit Wasser gespült, mit einem handelsüblichen Shampoo gewaschen und getrocknet. Die resultierenden Färbungen sind in Tabelle 1 zusammengefaßt.

Tabelle 1

| Beispiel | Entwicklersubstanz der Formel (I) | Kupplersubstanz | Farbe |
|---|---|---|---|
| 4 | 2,5-Diamino-benzonitril • HCl | 2-Amino-4-(2'-hydroxyethyl)amino-aniolsulfat | blau-violett |
| 5 | 2,5-Diamino-benzonitril • HCl | 5-Amino-2-methylphenol | hellrot |
| 6 | 5-Amino-2-methylamino-benzonitril • HCl | 2-Amino-4-(2'-hydroxyethyl)amino-anisolsulfat | dunkel-blau-violett |
| 7 | 5-Amino-2-methylamino-benzonitril • HCl | 5-Amino-2-methylphenol | rot |
| 8 | 5-Amino-2-(2'-hydroxyethyl)amino-benzonitril • HCl | 2-Amino-4-(2'-hydroxyethyl)amino-anisolsulfat | dunkel-blau-violett |
| 9 | 5-Amino-2-(2'-hydroxyethyl)amino-benzonitril • HCl | 5-Amino-2-methylphenol | rot |
| 10 | 5-Amino-2-(2'-hydroxyethyl)amino-benzonitril • HCl | m-Aminophenol | braun-rosé |
| 11 | 5-Amino-2-(2'hydroxyethyl)amino-benzonitril • HCl | Resorcin | dunkel-blond |
| 12 | 5-Amino-2-(2',3'-dihydroxypropylamino-benzonitril • HCl | 2-Amino-4-(2'hydroxyethyl)amino-anisolsulfat | dunkel-blau-violett |
| 13 | 5-Amino-2-(2',3'-dihydroxypropyl)amino-benzonitril • HCl | 5-Amino-2-methylphenol | rot |
| 14 | 2-Amino-2-(2'-hydroxyethyl)amino-benzonitril • HCl | 5-Amino-2-methylphenol | rosa |
| 15 | 2-Amino-2-(2',3'-dihydroxypropyl)amino-benzonitril • HCl | m-Aminophenol | blond-rosé |

**Beispiel 16:** Haarfärbemittel in Cremeform

| | |
|---|---|
| 3,33 g | 5-Amino-2-methylamino-benzonitril-Hydrochlorid |
| 1,50 g | Resorcin |
| 1,40 g | 2-Amino-4-(2'-hydroxyethyl)amino-anisolsulfat |
| 0,70 g | m-Aminophenol |
| 15,00 g | Cetylalkohol |
| 3,50 g | Natrium-laurylalkohol-diglykolethersulfat (28prozentige wäßrige Lösung) |
| 3,00 g | Ammoniak (25prozentige wäßrige Lösung) |
| 0,30 g | Natriumsulfit, wasserfrei |
| 71,27 g | Wasser |
| 100,00 g | |

50 g dieses Haarfärbemittels werden kurz vor Gebrauch mit 50 ml Wasserstoffperoxidlösung (6prozentig) vermischt. Anschließend trägt man das Gemisch auf blonde Naturhaare auf und läßt es 30 Minuten lang bei 40 Grad Celsius einwirken. Danach wird das Haar mit Wasser gespült und getrocknet. Das Haar hat eine schwarze Färbung erhalten.

**Beispiel 17:** Haarfärbemittel in Cremeform

| | |
|---|---|
| 0,07 g | 5-Amino-2-(2'-hydroxyethyl)amino-benzonitril-Hydrochlorid |
| 0,37 g | Resorcin |
| 0,11 g | 2-Methyl-resorcin |
| 0,04 g | 4-Amino-2-methylphenol |
| 0,07 g | 2-Amino-6-chlor-4-nitrophenol |
| 15,00 g | Cetylalkohol |
| 3,50 g | Natrium-laurylalkohol-diglykolethersulfat (28prozentige wäßrige Lösung) |
| 3,00 g | Ammoniak (25prozentige wäßrige Lösung) |
| 0,30 g | Natriumsulfit, wasserfrei |
| 77,54 g | Wasser |
| 100,00 g | |

50 g des obigen Haarfärbemittels werden kurz vor Gebrauch mit 50 ml Wasserstoffperoxidlösung (6prozentig) vermischt. Das Gemisch wird anschließend auf blonde Naturhaare aufgetragen und 30 Minuten bei 40 Grad Celsius einwirken gelassen. Nach dem Ausspülen mit Wasser und Trocknen hat das Haar eine modische goldbraune Farbe erhalten.

Alle Prozentangaben stellen, soweit nicht anders angegeben, Gewichtsprozente dar.

**Patentansprüche**

1. Mittel zur oxidativen Färbung von Haaren auf der Basis einer Entwicklersubstanz-Kupplersubstanz-Kombination, dadurch gekennzeichnet, daß es als Entwicklersubstanz ein 2,5-Diaminobenzonitril der allgemeinen Formel (I)

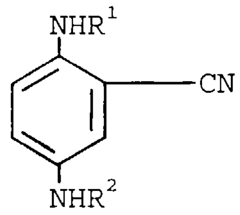

(I),

in der $R^1$ und $R^2$ unabhängig voneinander gleich Wasserstoff, Alkyl mit 1 bis 6 Kohlenstoffatomen oder Hydroxyalkyl mit 2 bis 4 Kohlenstoffatomen oder Dihydroxyalkyl mit 3 oder 4 Kohlenstoffatomen sind, unter der Voraussetzung, daß die beiden Reste $R^1$ und $R^2$ nicht gleichzeitig Wasserstoff darstellen, oder dessen physiologisch verträgliche, wasserlösliche Salze enthält.

2. Mittel nach Anspruch 1, dadurch gekennzeichnet, daß die Entwicklersubstanz der allgemeinen Formel (I) ausgewählt ist aus 5-Amino-2-methylamino-benzonitril, 5-Amino-2-(2'-hydroxyethyl)amino-benzonitril, 5-Amino-2-(2',3'-dihydroxypropyl)amino-benzonitril, 2-Amino-2-(2'-hydroxyethyl)amino-benzonitril, 2-Amino-2-(2',3'-dihydroxypropyl)amino-benzonitril und deren Hydrochloriden.

3. Mittel nach Anspruch 1 oder 2, dadurch gekennzeichnet, daß die Entwicklersubstanz der Formel (I) in einer Menge von 0,01 bis 5,0 Gewichtsprozent enthalten ist.

4. Mittel nach einem der Ansprüche 1 bis 3, dadurch gekennzeichnet, daß die Kupplersubstanz ausgewählt ist aus 1-Naphthol, Resorcin, 4-Chlorresorcin, 2-Methylresorcin, 2-Amino-4-(2'-hydroxyethyl)amino-anisol, 2,4-Diamino-5-ethoxytoluol, 4,6-Dichlorresorcin, m-Phenylendiamin, 5-Amino-2-methylphenol, 2,4-Diaminophenoxyethanol, 4-Amino-2-hydroxyphenoxyethanol, 3-Aminophenol, 3-Amino-2-methylphenol, 4-Hydroxy-1,2-methylendioxybenzol, 4-Amino-1,2-methylendioxybenzol, 4-(2'-Hydroxyethyl)amino-1,2,-methylendioxybenzol, 2,4-Diamino-5-fluortoluol, 3-Amino-4-chlor-6-methylphenol, 3-Amino-2-chlor-6-methylphenol, 3-Amino-4-fluor-6-methylphenol, 4-Hydroxyindol, 3-Amino-4-methoxy-6-methylphenol und 3,5-Diamino-2,6-dimethoxypyridin.

5. Mittel nach einem der Ansprüche 1 bis 4, dadurch gekennzeichnet, daß die Gesamtmenge der Entwicklersubstanz-Kupplersubstanz-Kombination 0,02 bis 10,0 Gewichtsprozent beträgt.

6. Mittel nach einem der Ansprüche 1 bis 5, dadurch gekennzeichnet, daß die Kupplersubstanz und die Entwicklersubstanz jeweils in einer Menge von 0,01 bis 5,0 Gewichtsprozent enthalten ist.

7. Mittel nach einem der Ansprüche 1 bis 6, dadurch gekennzeichnet, daß es eine zusätzliche Farbkomponente enthält, die ausgewählt ist aus 6-Amino-2-methylphenol, 2-Amino-5-methylphenol, 4-[(4'-aminophenyl)-(4'-imino-2",5"-cyclohexadien-1"-yliden)-methyl]-2-methyl-aminobenzol-monohydrochlorid (C.I. 42 510), 4-[(4'-amino-3'-methylphenyl)-(4"-imino-3"-methyl-2",5"-cyclohexadien-1"-yliden)-methyl]-2-methyl-aminobenzol-monohydrochlorid (C.I. 42 520), 4-(2'-Hydroxyethyl)amino-3-nitrotoluol, 2-Amino-4,6-dinitrophenol, 2-Amino-5-(2'-hydroxyethyl)amino-nitrobenzol, 1-[(2'-Ureidoethyl)amino]-4-nitrobenzol, 6-[(4'-Aminophenyl)azo]-5-hydroxy-naphthalin-1-sulfonsäure-Natriumsalz (C.I. 14 805), 1,4-Diamino-anthrachinon und 1,4,5,8-Tetraaminoanthrachinon.

8. 2,5-Diaminobenzonitrile der allgemeinen Formel (I)

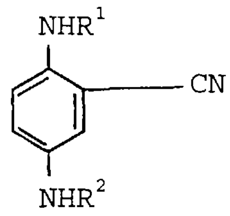

(I),

in der $R^1$ und $R^2$ unabhängig voneinander gleich Wasserstoff, Alkyl mit 1 bis 6 Kohlenstoffatomen oder Hydroxyalkyl mit 2 bis 4 Kohlenstoffatomen oder Dihydroxyalkyl mit 3 oder 4 Kohlenstoffatomen ist, oder dessen physiologisch verträgliche, wasserlösliche Salze.

**9.** 2-Amino-5-(2'-hydroxyethyl)amino-benzonitril.

**10.** 2-Amino-5-(2',3'-dihydroxypropyl)amino-benzonitril.

**11.** 5-Amino-2-methylamino-benzonitril.

**12.** 5-Amino-2-(2'-hydroxyethyl)amino-benzonitril.

**13.** 5-Amino-2-(2',3'-dihydroxypropyl)amino-benzonitril.

Europäisches Patentamt

# EUROPÄISCHER RECHERCHENBERICHT

Nummer der Anmeldung
EP 96 11 9746

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | US 5 139 532 A (CHAN A C ET AL) 18.August 1992<br>* Ansprüche; Beispiele 3,6 * | 1-13 | A61K7/13<br>C07C255/58 |
| A | US 5 034 014 A (WENKE GOTTFRIED) 23.Juli 1991<br>* Ansprüche * | 1-13 | |
| A | EP 0 299 497 A (KAO CORP) 18.Januar 1989<br>* Ansprüche; Tabelle 2 * | 1-13 | |
| A | EP 0 391 662 A (SQUIBB BRISTOL MYERS CO) 10.Oktober 1990<br>* Seite 4, Zeile 7 - Zeile 8; Ansprüche * | 1-13 | |
| A | WO 94 27564 A (OREAL ;LAGRANGE ALAIN (FR); VANDENBOSCHE JEAN JACQUES (FR); COTTER) 8.Dezember 1994<br>* Ansprüche * | 1-13 | |
| X | WO 93 13079 A (AGOURON PHARMACEUTICALS INC) 8.Juli 1993<br>* Seite 43, letzter Absatz *<br>* Seite 95, Zeile 12 - Zeile 24 * | 8 | RECHERCHIERTE SACHGEBIETE (Int.Cl.6)<br>A61K<br>C07C |
| X | DE 25 25 250 A (UPJOHN CO) 18.Dezember 1975<br>* Seite 14, Zeile 5 - Zeile 22 * | 8 | |

Der vorliegende Recherchenbericht wurde für alle Patentansprüche erstellt

| Recherchenort | Abschlußdatum der Recherche | Prüfer |
|---|---|---|
| DEN HAAG | 2.Juni 1997 | Seufert, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)